# EUROPEAN PATENT APPLICATION

(11) **EP 4 760 259 A1**
(43) Date of publication of application: **17.06.2026**
(21) Application number: 24307118.0
(22) Date of filing: 16.12.2024
(51) Int. Cl.: G01N 33/68

(54) **METHOD OF DETECTING POST-TRANSLATIONAL MODIFICATIONS OF CYSTEINE IN A PROTEIN OR POLYPEPTIDE**

(71) Applicant: Ecole Pratique des Hautes Etudes, 75014 Paris (FR)
(72) Inventor: PLENCHETTE, Stéphanie, 21560 COUTERNON (FR)
(74) Representative: Gevers & Orès

(57) **Abstract**

The present invention concerns a new method of detecting post-translational modification(s) of cysteine(s), in particular the S-nitrosylation, of a protein or polypeptide

## Description

The present invention concerns a new method of detecting post-translational modification(s) of cysteine(s), in particular the S-nitrosylation, of a protein or polypeptide.

Post-translational modifications (PTMs) of cysteine (Cys) residues serve many critical roles in regulating protein structure (e.g. tertiary structure), function, localization, and interactions within cells. Cys can be modified by oxidative reactions (e.g. S-nitrosylation, S-glutathionylation, S-sulfenylation) or by the attachment of lipid moieties (e.g. S-palmitoylation, S-farnesylation). Some oxidation of thiol groups can occur on Cys residues which can then form covalent disulfide bonds between Cys within the same protein or between proteins (intra- and intermolecular disulfide bonds, which are crucial for protein folding, stability, and structural integrity); while others are transient and reversible. Cys PTMs such as S-nitrosylation or S-sulfenylation are not catalyzed by specific enzymes and typically occur via direct reaction of thiols with reactive oxygen species (ROS), reactive nitrogen species (RNS) and other thiols.

In this context, there are key interests in studying these modifications.

Cysteine residues are highly susceptible to redox modifications which can regulate protein activity, stability, and signalling pathways. For instance, reversible oxidation of cysteine residues can modulate enzyme activity (e.g. by altering the catalytic properties or substrate specificity), gene expression, and cell signalling processes in response to changes in cellular redox status.

For example, reversible S-nitrosylation, S-sulfenylation, or S-glutathionylation of cysteine residues can regulate the activity of enzymes involved in various cellular processes, including metabolism, signalling, and stress response.

PTMs of cysteine residues serve as important signalling mechanisms in cellular communication. For instance, S-nitrosylation, S-palmitoylation, and S-glutathionylation of cysteine residues can regulate protein-protein interactions, subcellular localization, and downstream signalling events in response to various extracellular stimuli.

Dysregulation of cysteine PTMs is implicated in the pathogenesis of various diseases, including cancer, neurodegenerative disorders, and cardiovascular diseases. Studying aberrant cysteine modifications can provide insights into disease mechanisms, biomarker discovery, and potential therapeutic targets.

Targeting cysteine PTMs offers potential avenues for drug development and therapeutic interventions. It may therefore be advantageous to develop new small molecules or peptides that modulate cysteine modifications as therapeutic agents to regulate protein function, restore redox balance, or disrupt disease-associated signalling pathways.

Hence, studying post-translational modifications of cysteine residues is essential for understanding fundamental biological processes, disease mechanisms, and therapeutic strategies. These modifications regulate protein function, structure, signalling, and contribute to cellular homeostasis and adaptation to environmental changes.

In particular, protein S-nitrosylation, the covalent adduction of a nitroso group to a cysteine thiol side chain, has recently emerged as a main mechanism by which nitric oxide (NO) mediates a wide range of cellular functions and phenotypes. S-nitrosylation regulates diverse pathways such as G-protein-coupled receptor signalling, death receptor-mediated apoptosis, glutamate-dependent neurotransmission, vesicular trafficking, stimulation of prostaglandin synthesis, and the unfolded protein response. In addition, aberrant S-nitrosylation is implicated in disease states such as tumour initiation and growth, neurodegeneration and malignant hyperthermia.

Consequently, much effort is focused on understanding the role of S-nitrosylation in normal physiology and its contribution to pathophysiology. For example, several recent studies have shown that dysregulated S-nitrosylation of the ryanodine receptor (Ca2+-release channel) may contribute to cardiac arrhythmias, heart stroke and impaired exercise capacity.

As scientific interest in protein S-nitrosylation continues to intensify, an increasing number of studies are relying on biotin switch techniques (BST) for the detection of S-nitrosylated proteins (protein-SNOs) induced by NO released from NO donors or produced by NO synthases. This technique, also called biotin switch assay (BSA), relies on four major steps performed on cellular lysates. First of all, free thiol groups are blocked by methyl methanethiosulfate (MMTS), then ascorbate is used to reduce the bounds between NO moieties and Cys residues in target proteins. NO is removed and thiol groups newly revealed can then react with reactive biotin-HPDP to form a covalent bond (NO being exchange by biotin). In the last step, biotinylated proteins are purified by using NeutrAvidin resin. Identification of protein S-nitrosylated can be either performed by Mass Spectrometry analysis or by separating proteins by SDS-PAGE and analysed by Western blotting.

However, this technique that needs great amounts of cells in order to perform an analysis is not quantitative nor possible *in situ* in a protein specific way.

Accordingly, it is an object of the present invention to provide a method that can be carried out *in situ,* in particular in cells (and not in cell lysates only).

Another aim of the invention is to provide a method that enables to detect and quantify post-translational modifications of cysteine residues, in particular S-nitrosylation, of a protein or polypeptide.

Another aim of the invention is to provide a method that needs only a small quantity of cells for the analysis, for example up to 25 times less compared to the prior art techniques.

Thus, the present invention relates to a method of detecting post-translational modification(s) of cysteine(s) of a protein or polypeptide, said method comprising the following steps:
(i) A step of blocking said protein or polypeptide with a free thiol-blocking agent, to obtain a free thiol-blocked protein or polypeptide;
(ii) A step of reducing the post-translational modified thiol of cysteine residue(s) of the free thiol-blocked protein or polypeptide obtained in step (i), if any, in thiol group(s);
(iii) A step of biotinylation of the thiol group(s), if any;
(iv) A step of detecting the biotinylation obtained in step (iii) by Proximity Ligation Assay (PLA).

By "polypeptide" is in particular meant a chain of amino acids linked by peptide bonds containing from 10 to 100 amino acids; above 100 amino acids, the chain being referred to as a protein.

In a particular embodiment, the post-translational modification of cysteine is S-nitrosylation, S-glutathionylation, S-sulfenylation, S-Sulfinylation, S-Sulfonylation S-sulfhydrylation (persulfidation), S-palmitoylation, S-prenylation, S-cyanylation, S-acylation, S-carbonylation, S-itaconation, S-succination, and/or S-CoAlation.

In a more particular embodiment, the method of the invention, of detecting the S-nitrosylation of a protein or polypeptide, comprises the following steps:
(i) A step of blocking said protein or polypeptide with a free thiol-blocking agent, to obtain a free thiol-blocked protein or polypeptide;
(ii) A step of reducing the S-NO group(s) of the free thiol-blocked protein or polypeptide obtained in step (i), if any, in thiol group(s);
(iii) A step of biotinylation of the thiol group(s), if any;
(iv) A step of detecting the biotinylation obtained in step (iii) by Proximity Ligation Assay (PLA).

In a particular embodiment, said method is an *in vitro* method, in particular an *in cellulo* method, or an *in situ* method, in particular an *ex vivo* method, more particularly on a tissue.

In a more particular embodiment, said method is an *in cellulo* method.

In an even more particular embodiment, steps (i) to (iv) are conducted on an amount of cells comprised from 10⁵ to 5.10⁶ cells, in particular, from steps (i) to (iii) on an amount of cells comprised from 1.10⁶ to 5.10⁶ cells and in step (iv) on an amount of cells comprised from 10⁵ to 5.10⁵ cells.

In another even more particular embodiment, step (i) is conducted away from light.

In another even more particular embodiment, step (i) (in particular conducted away from light) is preceded by one step of fixation and optionally permeabilization of the cells (in particular realized away from light). The step(s) of fixation and optionally permeabilization may be realized, or not, depending on whether the protein of interest is localized intracellularly. In another even more particular embodiment, step (i) is not preceded by one step of fixation and optionally permeabilization of the cells, in particular if the protein of interest is localized at the plasma membrane.

Thus, for transmembrane protein analysis on its extracellular domains, step (i) may not be preceded by one step of fixation and optionally permeabilization.

In consequence, step (i) is preceded or not by one step of fixation, with or without permeabilization.

In a particular embodiment, methanethiosulfonate (MMTS) is used for blocking free thiol of step (i).

In a particular embodiment, step (i) is conducted for 10 to 60 minutes, in particular for about 20 minutes, and/or at a temperature comprised from 40 to 60°C, notably from 48°C to 52°C in particular at about 50°C.

In a particular embodiment, step (i) is realized away from light.

In a particular embodiment, the free thiol-blocking agent of step (i) is used at a concentration from 1 to 200 mM in particular about 20 mM, in a solution, in particular in a blocking buffer. Said blocking buffer is for example MMTS (Sigma-Aldrich, product No. 64306) in HEN buffer.

In a particular embodiment, step (ii) is performed by contacting free thiol-blocked protein or polypeptide obtained in step (i) with a reducing compound, in particular chosen from ascorbic acid and dithiothreitol (DTT), preferably ascorbic acid.

In a particular embodiment, step (ii) is realized away from light.

In a particular embodiment, the reducing agent of step (ii) is used at a concentration from 0.1 to 10 mM, in particular about 1 mM, in a solution, in particular in a buffer.

In a particular embodiment, step (iii) is performed by using a biotinylation agent, in particular biotine-HPDP (N-[6-(biotinamido)hexyl]-3'-(2'-pyridyldithio)propionamide).

In a particular embodiment, the biotinylation agent of step (iii) is used at a concentration from 0.01 to 10 mM, in particular from 0.1 to 1 mM, preferably about 0.4 mM, in a solution, in particular in a buffer.

In a particular embodiment, steps (ii) and (iii) are concerted.

In a particular embodiment, steps (ii) and (iii) are conducted for 30 minutes to two hours, in particular for about 1 hour, and/or at a temperature comprised from 18°C to 30°C, in particular at about 20°C.

In a particular embodiment, step (ii) is performed by contacting free thiol-blocked protein or polypeptide obtained in step (i) with a reducing agent, and wherein step (iii) is performed by using a biotinylation agent, said reducing agent and said biotinylation agent being present in a same solution, in particular a same buffer.

In a particular embodiment, step (i) is preceded or not by one step of fixation with or without permeabilization, and one or more washing steps are realized, in particular with a buffer, for example with a Biotin Switch Assay (BSA) buffer or a phosphate-buffered saline (PBS) buffer:
- After the optional fixation and optionally permeabilization step(s), and prior to step (i); and /or
- Between step (i) and (ii); and /or
- Between step (ii) and (iii); and /or
- Between step (iii) and (iv).

In a particular embodiment, step (iv) of detecting biotinylation is performed with a flow cytometry readout.

In another particular embodiment, step (iv) of detecting biotinylation is performed with a fluorescence spectroscopy or microscopy readout.

In a particular embodiment, said method is an *ex vivo* method, in particular on a tissue, step (iv) of detecting biotinylation being performed with a fluorescence or microscopy spectroscopy readout.

In a particular embodiment, step (iv) of detecting biotinylation comprises the following substeps:
a) Binding two primary antibodies, in particular raised in different species, recognizing said protein or polypeptide, and biotin respectively;
b) Binding two probes, the first probe comprising a single oligonucleotide trail conjugated to a secondary antibody directed against the first primary antibody, for example against the constant regions of said first primary antibody, the second probe comprising a single oligonucleotide trail conjugated to a secondary antibody directed against the second primary antibody, for example against the constant regions of said second primary antibody;
c) ligating the first and second oligonucleotide (PLUS and MINUS probe pair) tails thereby producing a ligated DNA template; and
d) performing amplification of the DNA template across the first and second oligonucleotides.

### FIGURES

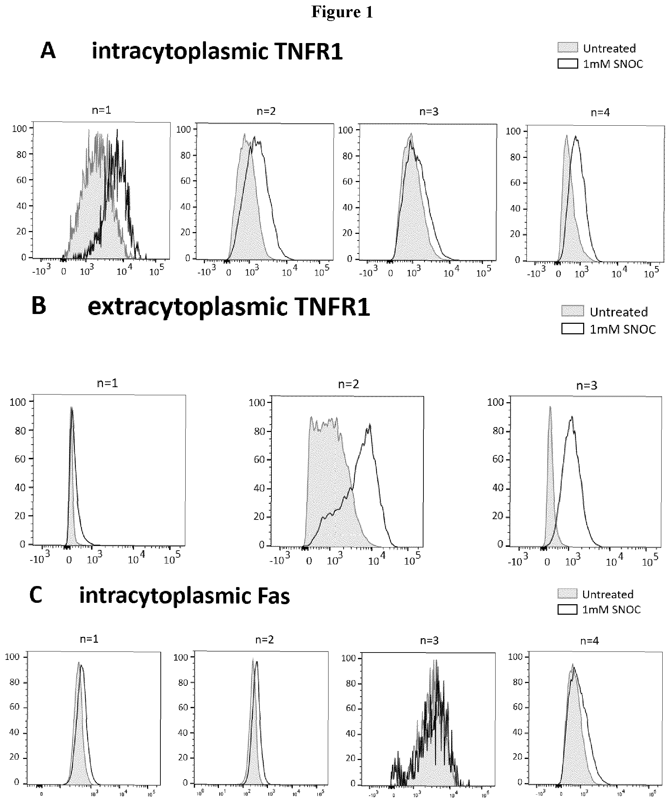

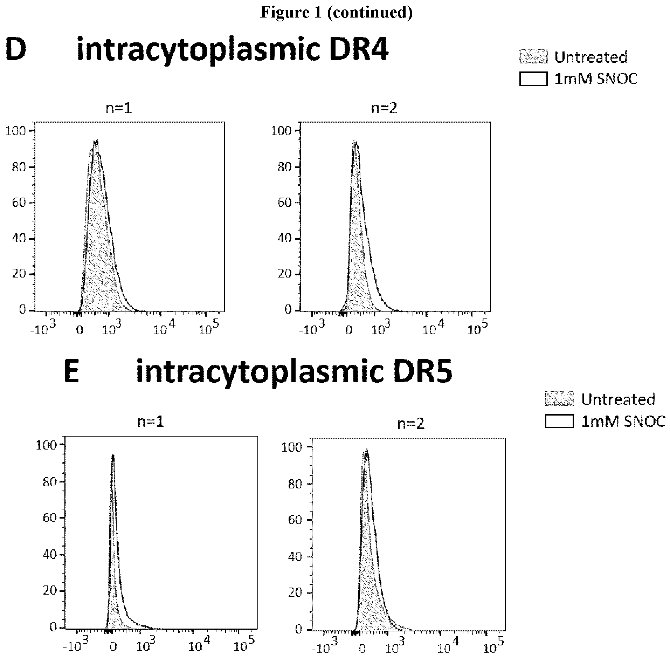

**Figure 1** presents results obtained on Colorectal Adenocarcinoma Cells (SW480), using S-nitrosocysteine (SNOC) as NO donor; and **A:** Anti-TNFR1 *(Euromedex ; HB-HA500140),* Anti-biotine *(Biolegend; 409002);* **B:** Anti-TNFR1 *(Biorbyt ; orb100329),* Anti-biotine *(Biolegend ; 409002);* C: Anti-Fas *(Invitrogen; MA5-32489),* Anti-biotine *(Biolegend; 409002) ;* **D:** Anti-DR4 *(Cell Signaling ; 42533S),* Anti-biotine *(Biolegend; 409002) ;* **E:** Anti-DR5 *(Ozyme ; ABC-A19043),* Anti-biotine *(Biolegend; 409002).* n indicates the number of experiment performed.

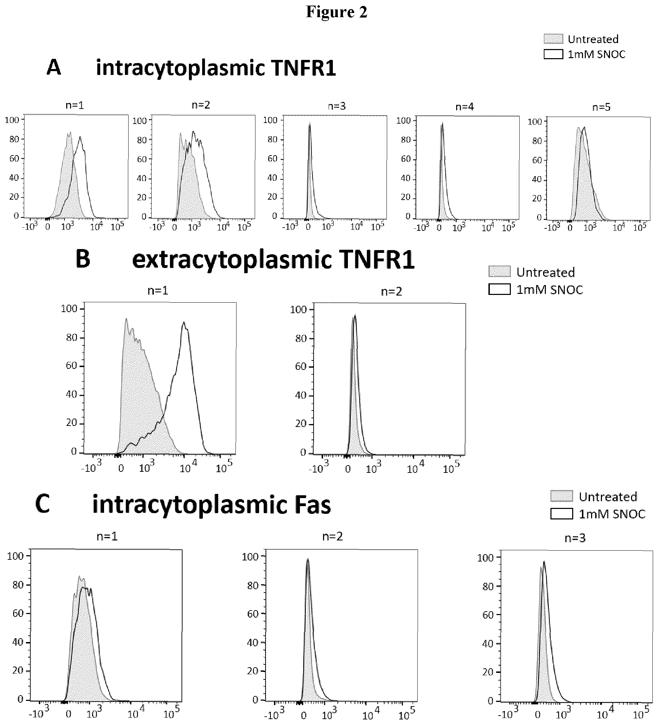

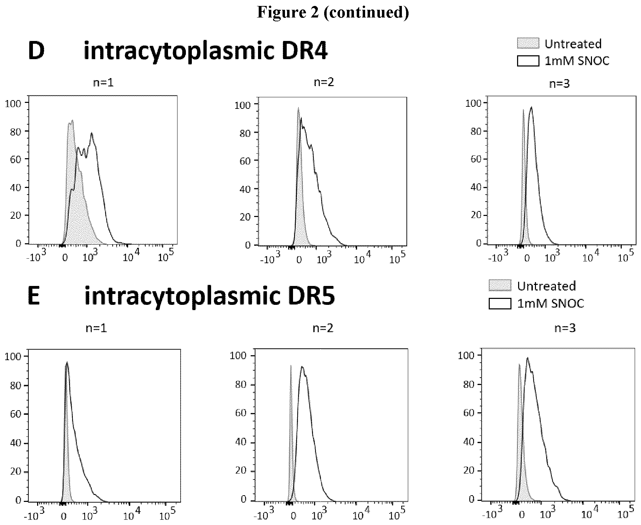

**Figure 2** presents results obtained on Colorectal Adenocarcinoma Cells (SW620), using S-nitrosocysteine (SNOC) as NO donor; and **A:** Anti-TNFR1 *(Euromedex ; HB-HA500140),* Anti-biotine *(Biolegend; 409002);* **B:** Anti-TNFR1 *(Biorbyt ; orb100329),* Anti-biotine *(Biolegend ; 409002);* **C:** Anti-Fas *(Invitrogen; MA5-32489),* Anti-biotine *(Biolegend; 409002) ;* **D:** Anti-DR4 *(Cell Signaling ; 42533S),* Anti-biotine *(Biolegend; 409002) ;* **E:** Anti-DR5 *(Ozyme ; ABC-A19043),* Anti-biotine *(Biolegend; 409002).* n indicates the number of experiment performed.

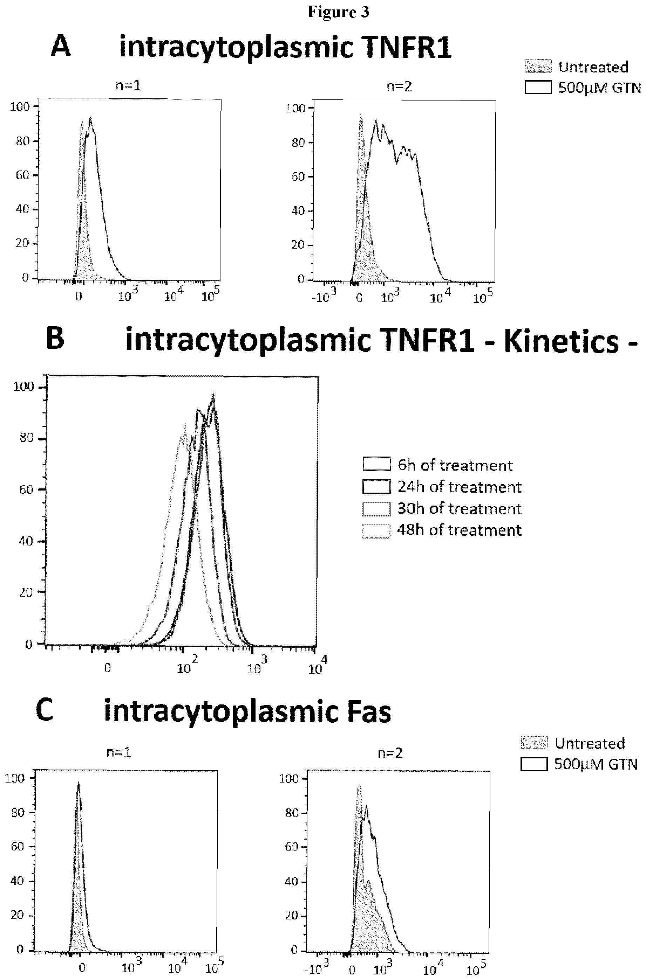

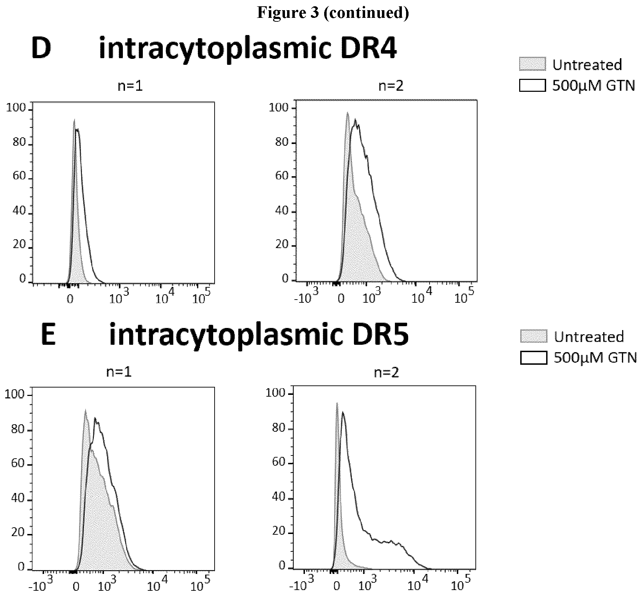

**Figure 3** presents results obtained on Colorectal Adenocarcinoma Cells (SW620), using Glyceryl Trinitrate (GTN) as NO donor; and **A:** Anti-TNFR1 *(Euromedex ; HB-HA500140),* Anti-biotine *(Biolegend ; 409002);* **B:** same as **A,** kinetics; **C:** Anti-Fas *(Invitrogen ; MA5-*32489), Anti-biotine *(Biolegend; 409002) ;* **D:** Anti-DR4 *(Cell Signaling; 42533S),* Anti-biotine *(Biolegend; 409002) ;* **E:** Anti-DR5 *(Ozyme ; ABC-A19043),* Anti-biotine *(Biolegend ; 409002).* n indicates the number of experiment performed.

### EXAMPLES

### Example 1: Protocol for intracellular analysis of S-nitrosylation according to the invention

Variations to the protocol where made when indicated for the analysis of transmembrane protein S-nitrosylation on the extracellular side.
1. Treatment : seed and treat 2×10⁶ cells with or without an NO donor protected from light as indicated right below.
   - SNOC treatment: 1 mM for 15 min.
   - Nitronal (GTN) treatment: 500 µM for 6-48h.
   The samples are protected from light from steps 1 to 6.
   All required dilutions are performed in high purity water.
2. Collect and centrifuge the samples at 400 g for 5 min at 4°C. Wash the samples two times in 1x phosphate-buffered saline (PBS) and remove residual 1x PBS from cells.
3. Fixation and permeabilization : resuspend cells in 100 µL of BD Cytofix/Cytoperm^{™} solution (BD Biosciences, product No. 554714) to fix and permeabilize samples and incubate for 20 min at 4°C. For analysis of transmembrane protein S-nitrosylation on extracellular domains 1) skip this step and proceed to step 4, and 2) perform all washes from steps 4 to 15 with 1x PBS containing 0.4 mM EDTA and 0.04 mM Neocuproine.
4. Following fixation and permeabilization, wash the samples two times in BSA wash buffer and remove residual wash buffer from cells. BSA wash buffer consists of 1x BD Perm/Wash^{™} buffer (BD Biosciences, product No. 554723) containing 0.4 mM EDTA and 0.04 mM Neocuproine.
5. Blocking free thiols : resuspend cells in 1 mL of blocking buffer (prepared immediately prior use) and incubate by mixing samples for 20 min at 50°C to block free thiols.
   It is recommended to use an Eppendorf ThermoMixer^{®} for heating and mixing samples. Otherwise, the tubed must be gently inverted at least 4 times to ensure proper mixing. HEN buffer (250 mM HEPES pH 7.7, 1 mM EDTA, 0.1 mM Neocuproine).
   Blocking buffer (20 mM MMTS (Sigma-Aldrich, product No. 64306) in HEN buffer).
6. Following blocking free thiols, wash the samples two times in BSA wash buffer and remove residual wash buffer from cells. BSA wash buffer consists of 1x BD Perm/Wash^{™} buffer (BD Biosciences, product No. 554723) containing 0.4 mM EDTA and 0.04 mM Neocuproine.
7. Biotinylation : resuspend cells in 1 mL of HEN buffer containing 1 mM ascorbate and 0.4 mM of HPDP-biotin (EZ-Link^{®} HPDP-biotin, ThermoFisher Scientific, product No. 21341) and incubate for 1h at room temperature using an orbital shaker to mix the samples. HPDP-biotin can be omitted from some samples as a control. HEN buffer (250 mM HEPES pH 7.7, 1 mM EDTA, 0.1 mM Neocuproine).
8. Following biotinylation, wash the samples two times in 1x PBS and remove residual wash buffer from cells.
9. Blocking : resuspend cells in 30 µL of blocking solution (≈ 1 drop of the Duolink^{®} Blocking Solution) and incubate for 1h at 37°C. Aliquot 20 µL of each sample cells in final volume 100 µL 1x PBS per well of a V-bottom plate. Centrifuge the plate at 400 *g* for 5 min at room temperature and remove the Duolink^{®} Blocking Solution from the cells.
   The Duolink^{®} blocking solution is provided with the Duolink^{®} PLA probes (Sigma-Aldrich).
10. Primary antibody incubation : add 100 µL of the primary antibodies solution to each sample and mix well. Incubate the plate overnight at 4°C. Dilute primary antibodies to suitable concentration in the Duolink^{®} Antibody Diluent provided with the Duolink^{®} PLA probes.
   One antibody is directed against the protein of interest and the second one is directed against biotin. It is required that the two primary antibodies come from different host species.
11. Duolink^{®} PLA Probe incubation : centrifuge the plate at 400 g for 5 min at room temperature and remove the solution from the cells. Wash the cells twice with 200 µL of prepare Duolink^{®} Wash Buffer per well, centrifuge at 400 g for 5 min at room temperature, and remove the solution from the cells. Add 100 µL of the PLA probe solution and mix well. Incubate in a 37 °C incubator for 1 hour.
   To prepare the PLA probe solution, dilute the PLUS and MINUS PLA probes 1:5 in the Duolink^{®} Antibody Diluent. For a 100 µL reaction, take 20 µL of PLA probe MINUS stock, 20 µL of PLA probe PLUS stock and 60 µL of Antibody Diluent. Make sufficient solution for all samples.
   Duolink^{®} Wash Buffer should be made prior to beginning the assay by dissolving the contents of one pouch in high purity water to a final volume of 1000 mL. Wash buffer may be stored at room temperature for short term storage (less than two weeks) or at 4 °C for long term storage.
12. Ligation : centrifuge the plate at 400 g for 5 min at room temperature and remove the solution from the cells. Wash the cells twice with 200 µL of Duolink^{®} Wash Buffer (Sigma-Aldrich) per well, centrifuge at 400 g for 5 min at room temperature, and remove the solution from the cells. Add 100 µL of ligation solution for each sample cells and mix well. Incubate in a 37 °C incubator for 30 min.
   Vortex the 5x Duolink^{®} Ligation buffer. Dilute the 5x Ligation buffer 1:5 in high purity water and mix. For a 100 µL reaction, add 20 µL of the 5x Ligation buffer to 77.5 µL of high purity water. Make sufficient solution for all samples.
   During the wash, retrieve the Ligase from the freezer using a freezer block (-20 °C). Add Ligase to the 1x Ligation buffer at a 1:40 dilution and mix. For 100 µL ligation solution, add 2.5 µL of Ligase to 97.5 µL of the 1x ligation buffer.
   Duolink^{®} Wash Buffer should be made prior to beginning the assay by dissolving the contents of one pouch in high purity water to a final volume of 1000 mL. Wash buffer may be stored at room temperature for short term storage (less than two weeks) or at 4 °C for long term storage.
13. Amplification : centrifuge the plate at 400 g for 5 min at room temperature and remove the solution from the cells. Wash the cells twice with 200 µL of Duolink^{®} Wash Buffer per well, centrifuge at 400 g for 5 min at room temperature, and remove the solution from the cells. Add 100 µL of amplification solution for each sample cells and mix well. Incubate in a 37 °C incubator overnight.
   Wait to add the polymerase until immediately prior to addition to the sample. Vortex the 5x Duolink^{®} Amplification buffer. Dilute the 5x Amplification buffer 1:5 in high purity water and mix. For 100 µL reaction, add 20 µL of the 5x Amplification buffer to 78.75 µL of high purity water. Make sufficient solution for all samples. During the wash, retrieve the Polymerase from the freezer using a freezer block (-20 °C). Add Polymerase to the 1x Amplification buffer at a 1:80 dilution and mix. For 100 µL amplification solution, add 1.25 µL of Polymerase to 98.75 µL of the 1x amplification buffer.
   Amplification times can be adjusted (from 100 minutes to overnight) depending on protein abundance or protein interactions.
   Duolink^{®} Wash Buffer should be made prior to beginning the assay by dissolving the contents of one pouch in high purity water to a final volume of 1000 mL. Wash buffer may be stored at room temperature for short term storage (less than two weeks) or at 4 °C for long term storage.
14. Detection : centrifuge the plate at 400 g for 5 min at room temperature and remove the solution from the cells. Wash the cells twice with 200 µL of Duolink^{®} Wash Buffer per well, centrifuge at 400 g for 5 min at room temperature, and remove the solution from the cells. Add 100 µL of Duolink^{®} Detection Buffer solution and mix well. Incubate in a 37 °C incubator for 60 minutes.
   The Duolink^{®} Detection Buffer is light-sensitive. Protect from light. Vortex the 5x Detection Buffer. Dilute 1:5 in high purity water and mix. For 100 µL reaction, add 20 µL of the 5x Detection buffer to 80 µL of high purity water. Make sufficient solution for all samples. Detection times can be adjusted (from 10-60 minutes) depending on protein abundance or level of background.
   Duolink^{®} Wash Buffer should be made prior to beginning the assay by dissolving the contents of one pouch in high purity water to a final volume of 1000 mL. Wash buffer may be stored at room temperature for short term storage (less than two weeks) or at 4 °C for long term storage.
15. Final washes : centrifuge the plate at 400 g for 5 min at room temperature and remove the solution from the cells. Wash the cells with 200 µL of Duolink^{®} Wash Buffer per well, centrifuge at 400 g for 5 min at room temperature, and remove the solution from the cells. Resuspend the cells in 300 µL 1x PBS and transfer to a flow cytometer tube. Use a flow cytometer with appropriate filters and software for data analysis.
   Duolink^{®} Wash Buffer should be made prior to beginning the assay by dissolving the contents of one pouch in high purity water to a final volume of 1000 mL. Wash buffer may be stored at room temperature for short term storage (less than two weeks) or at 4 °C for long term storage.

### Example 2: Results

The protocol that was used here is that described above in example 1. The details of the dilution of primary antibodies for the detection of S-nitrosylation (SNO) of the various proteins are given below:
SNO-TNFR1: anti-Biotin (mouse) 1/200 - anti-TNFR1 (rabbit) 1/500
SNO-DR4: anti-Biotin (mouse) 1/200 - anti-DR4 (rabbit) 1/50
SNO-DR5: anti-Biotin (mouse) 1/200 - anti-DR5 (rabbit) 1/100
SNO-Fas : anti-Biotin (mouse) 1/200 - anti-Fas (rabbit) 1/50

Results are presented in figures 1-3.

Overall, these results indicated that the presents method can detect specific S-nitrosylated proteins *in situ.* Figures 1-3 provided evidence that the S-nitrosylation of transmembrane receptors can be detected specifically on either side of the plasma membrane. Hence this method brings advantages for the analysis of the S-nitrosylation of proteins in a spacio-specific manner.

## Claims

1. A method of detecting post-translational modification(s) of cysteine(s) of a protein or polypeptide, said method comprising the following steps:
(i) A step of blocking said protein or polypeptide with a free thiol-blocking agent, to obtain a free thiol-blocked protein or polypeptide;
(ii) A step of reducing the post-translational modified thiol of cysteine residue(s) of the free thiol-blocked protein or polypeptide obtained in step (i), if any, in thiol group(s);
(iii) A step of biotinylation of the thiol group(s), if any;
(iv) A step of detecting the biotinylation obtained in step (iii) by Proximity Ligation Assay (PLA).

2. The method according to claim 1, wherein the post-translational modification of cysteine is S-nitrosylation, S-glutathionylation, S-sulfenylation, S-Sulfinylation, S-Sulfonylation S-sulfhydrylation (persulfidation), S-palmitoylation, S-prenylation, S-cyanylation, S-acylation, S-carbonylation, S-itaconation, S-succination, and/or S-CoAlation.

3. The method according to anyone of the preceding claims, wherein said method is an *in vitro* method, in particular an *in cellulo* method, or an in situ method, in particular an *ex vivo* method, more particularly on a tissue.

4. The method according to anyone of the preceding claims, wherein said method is an *in cellulo* method, and in particular wherein steps (i) to (iv) are conducted on an amount of cells comprised from 10⁵ to 10⁷ cells, in particular 1.10⁶ to 4.10⁶ cells, more particularly about 2.10⁶ cells.

5. The method according to claim 4, wherein step (i) is preceded by one step of fixation and optionally permeabilization of the cells, realized away from light.

6. The method according to anyone of the preceding claims, wherein the free thiol-blocking agent of step (i) is methanethiosulfonate (MMTS), said step (i) being in particular realized away from light.

7. The method according to anyone of the preceding claims, wherein step (ii) is performed by contacting free thiol-blocked protein or polypeptide obtained in step (i) with a reducing agent, in particular chosen from ascorbic acid and dithiothreitol (DTT), preferably ascorbic acid.

8. The method according to anyone of the preceding claims, wherein step (iii) is performed by using a biotinylation agent, in particular biotine-HPDP (N-[6-(biotinamido)hexyl]-3'-(2'-pyridyldithio)propionamide).

9. The method according to anyone of the preceding claims, wherein step (ii) is performed by contacting free thiol-blocked protein or polypeptide obtained in step (i) with a reducing agent, and wherein step (iii) is performed by using a biotinylation agent, said reducing agent and said biotinylation agent being present in a same solution, in particular a same buffer.

10. The method according to anyone of claims 4 to 9, wherein step (i) is preceded by one step of fixation and optionally permeabilization, and wherein one or more washing steps are realized, in particular with a buffer, for example with a Biotin Switch Assay (BSA) buffer or a phosphate-buffered saline (PBS) buffer:
- After the fixation and optionally permeabilization steps, and prior to step (i); and /or
- Between step (i) and (ii); and /or
- Between step (ii) and (iii); and /or
- Between step (iii) and (iv).

11. The method according to anyone of the preceding claims, wherein step (iv) of detecting biotinylation is performed with a flow cytometry readout.

12. The method according to anyone of the claims 1 to 10, wherein step (iv) of detecting biotinylation is performed with a fluorescence microscopy readout.

13. The method according to claim 12, wherein said method is an *ex vivo* method, in particular on a tissue, step (iv) of detecting biotinylation being performed with a fluorescence microscopy readout.

14. The method according to anyone of the preceding claims, wherein step (iv) of detecting biotinylation comprises the following substeps:
a) Binding two primary antibodies, in particular raised in different species, recognizing said protein or polypeptide, and biotin respectively;
b) Binding two probes, the first probe comprising a single oligonucleotide trail conjugated to a secondary antibody directed against the first primary antibody, for example against the constant regions of said first primary antibody, the second probe comprising a single oligonucleotide trail conjugated to a secondary antibody directed against the second primary antibody, for example against the constant regions of said second primary antibody;
c) ligating the first and second oligonucleotide (PLUS and MINUS probe pair) tails thereby producing a ligated DNA template; and
d) performing amplification of the DNA template across the first and second oligonucleotides.
